(19) Europäisches Patentamt

European Patent Office

Office européen des brevets

(11) **EP 4 461 212 A1**

(12) **EUROPEAN PATENT APPLICATION**

(43) Date of publication:
**13.11.2024 Bulletin 2024/46**

(21) Application number: **23172461.8**

(22) Date of filing: **09.05.2023**

(51) International Patent Classification (IPC):
**A61B 5/026** (2006.01)    **A61B 5/374** (2021.01)

(52) Cooperative Patent Classification (CPC):
**A61B 5/026; A61B 5/374; A61B 5/4064**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
• **Assistance Publique Hôpitaux de Paris**
**75012 Paris (FR)**

• **Institut National de Recherche en Informatique et en Automatique**
**78150 Rocquencourt (FR)**

(72) Inventor: **The designation of the inventor has not yet been filed**

(74) Representative: **Flesselles, Bruno F.G.**
**BF IP**
**36 rue Jean de la Fontaine**
**75016 Paris (FR)**

(54) **METHOD FOR DETECTING BRAIN HYPOPERFUSION DURING ANESTHESIA**

(57)    The invention relates to the field of anesthesia and of monitoring of the patient's state during anesthesia, notably the blood perfusion of the brain, using data from the electroencephalogram of the patient.

**Figure 1**

**Description**

The invention relates to the field of anesthesia and of monitoring of the patient's state during anesthesia, notably the blood perfusion of the brain.

Under general anesthesia (GA), maintaining optimal cerebral perfusion is crucial to ensure organ function, especially the brain. The conventional practice is to monitor perioperative mean arterial pressure (mAP) and verifying that it remains at an adequate value, although it is now considered that setting individualized mAP levels may better preserve cerebral perfusion. One potential strategy to determine such individualized mAP levels is to implement pharmacologically induced mAP changes (mAP challenge) and examine the relationship between mAP and cerebral blood flow (CBF), for instance measured with a transcranial Doppler exam.

One commonly accepted view of the blood pressure/cerebral blood flow relationship is based on the Lassen curve that assumes that there is an optimal range of cerebral perfusion, mAP typically between 60 and 150 mmHg, where the cerebral blood flow is maintained at a relatively constant level. However, such assumption of a wide cerebral autoregulation plateau is incorrect and is greatly variable between individuals (Brassard et al, Physiol Rep. 2021 Aug; 9(15): e14982, doi: 10.14814/phy2.14982).

It is now admitted that transient changes in mAP during anesthesia can affect cerebral perfusion. However, in practice, the relationship between mAP and CBF remains poorly explored. Indeed, mAP is not an accurate enough gold standard proxy for cerebral blood flow Some patients are indeed able to compensate for cerebral blood flow in the event of a drop in mAP, while others don't have this ability. Furthermore, current methods for monitoring cerebral perfusion, such as near-infrared spectroscopy (NIRS) and transcranial Doppler, have limitations in their applicability and effectiveness, necessitating alternative approaches.

Intraoperative frontal electroencephalogram (EEG) monitoring offers several advantages as a potential tool for assessing cerebral perfusion in the context of general anesthesia. Routinely used in clinical practice for assessing depth-of-anesthesia, EEG provides high temporal resolution data, allowing for rapid analysis of brain activity. However, it has not yet been validated whether EEG can be used to monitor CBF and the potential delay between changes in the CBF and modifications in the EEG signal.

The impact of cerebral perfusion on EEG signals has been suggested in pathological situations for prolonged brain hypoperfusion, such as the ones observed after carotid clamp, cardiac arrest, or stroke. In these conditions, variations in CBF can impact EEG. Nevertheless, the relationship between CBF and EEG has not been adequately investigated in the context of general anesthesia and in dynamic conditions where CBF variations occur rapidly, over a minute, for instance following a transitory or prolonged mAP variation.

The inventors addressed this issue and established the utility of EEG as a tool for monitoring cerebral perfusion by demonstrating a statistically significant linear relationship between CBF changes induced by a mAP challenge and intraoperative EEG.

They also determined the lag that maximizes the relationship between actual variation of the CBF (hypoperfusion) and variation of the EEG.

The continuous monitoring of cerebral perfusion in the context of anesthesia, through the use of the EEG signal, developed by the inventors through the methods and systems herein disclosed, makes it possible to contribute to the development of more individualized, effective and accessible strategies for maintaining cerebral perfusion during general anesthesia. The methods use information from the alpha brain waves (neural oscillations in the frequency range of 7.5-13 Hz (or 8-13 Hz, or 8-12 Hz, depending on the authors) which are reduced with open eyes and sleep and enhanced during drowsiness) and delta brain waves (neural oscillations with a frequency between 0.5 and 4 hertz, usually associated with the deep stage 3 of sleep, and which could be used for characterizing the depth of sleep).

The inventors showed that it is possible to detect a drop in the CBF by using a signal extracted from an EEG (which is routinely used during anesthesia), and that the delay between the decrease of brain perfusion and the appearance of the signal is about 5-7 minutes (between 300-420 seconds, especially between 350-400 seconds). Although this delay may seem to be important, it is in line with the practice of anesthetists during general anesthesia, in particular when such anesthesia are long ones (more than 30 minutes).

The invention thus relates to an ex *vivo* method for continuously monitoring brain perfusion during anesthesia of a subject, wherein the patient is in a state of stable anesthesia, comprising

    a. Receiving, at consecutive time points, data from an electroencephalogram (EEG) of the subject, wherein this data comprises the cerebral activity recorded during a time window preceding a given time point
    b. Extracting alpha and delta waves frequency information
    c. Calculating delta / alpha ratio (DAR) using the information of b), wherein the DAR is the ration of the power of the alpha and delta waves
    d. Comparing the calculated DAR to the DAR calculated at the preceding time point,

wherein increase of the calculated DAR indicates hypoperfusion of the brain of the subject.

The method may be automized. In view of the steps of the methods, it appears that at least some steps (notably the extraction of the alpha and delta waves frequency information) are implemented by a computer. Preferably all steps are implemented by a computer and the method would thus be considered as being computer-implemented. It is also to be noted that the method is performed ex vivo. Even though it uses EEG data, the method doesn't include the steps of measuring the EEG of the subject, but only the steps of processing the EEG data.

The method is performed with the data of a subject that is in a stable (or stationary) anesthetic state. The anesthesia is considered as stable/stationary when the rate of anesthetic drug administered to the patient is stable (and thus when the blood concentration of anesthetic drugs is stable. It is reminded that the anesthetist uses a pharmacokinetic model (Schinder model) to identify the stable rate of anesthetic drug to use, taking into account that there is a small adaptation time when the dose is changed. According to this model, the physician seeks a stable concentration in the brain, the target concentration being determined by the anesthetist prior to the start of the anesthesia, and according to the patient's profile (age, weight, Body Mass Index, presence of comorbidities...).

The Schinder model is integrated in devices already used in the operating room. Such devices comprise processors that calculates the amount of anesthetic drug to inject to the patient (the rate of injection) to obtain the target concentration that has been set up by the anesthetist, using a processor-controlled syringe pump. A monitor with a screen makes it possible for the physician to see the blood theoretical concentration (which is calculated according to the model, depending on the amount of propofol injected).

Generally, the anesthesia is stable about 5-10 minutes after induction of anesthesia. As an illustration, when the anesthetist selects a propofol target concentration of 3.5 ng/ml, induction is performed with a higher amount (5 ng/ml) and the rate of propofol administration is then lowered to reach the target concentration. The physician will enter the induction amount and the target concentration in the dispensing device, which calculates the various rates of propofol administration to reach these requested amounts. Although propofol has been herein cited, other anesthetic drugs such as benzodiazepines, diazepam, lorazepam, midazolam, etomidate, ketamine, which are also administered intravenously, can be used. It is preferred to perform the method with a subject that has been subjected to an anesthesia through intravenous administration of a sedative drug.

Propofol is the most commonly used intravenous general anesthetic. At low doses, it induces sleep while allowing the patient to continue breathing on his or her own. At higher doses, propofol is a respiratory depressant. It can be used to induce unconsciousness for intubation (placement of a breathing tube) and other surgical procedures. The method is of great interest when propofol is used.

The method first requires receiving, at consecutive time points, data from an electroencephalogram (EEG) of the subject. Such data comprises the cerebral activity recorded during a time window preceding a given time point.

The method being intended to have a monitoring of the brain perfusion during the anesthesia, the steps are performed on a regular basis (the different consecutive time points), and the result obtained at one time point is compared with the result obtained at the preceding time point.

Since the EEG data of the patient is to be processed to extract information pertaining to the alpha and delta brain waves, there is a need to acquire data on a time window sufficiently long to allow processing of such data and extraction of the needed information. The EEG data that is used in the method is thus preferably acquired on a time comprised between 5 and 30 seconds, preferably between 5 and 20 seconds, preferably between 5 and 15 seconds. A time window of between 10 and 15 seconds, in particular of 10 seconds is adequate.

It is preferred when the time points on which the method is performed are spaced apart by a duration corresponding to the time window. They can be spaced apart by a longer duration (for instance two or three times the time window). However, using such spacing between two consecutive time points where the calculations are performed (the time window needed for proper calculation) makes it possible to obtain a continuous monitoring of the brain perfusion (this is continuous in the sense that there is no interval during which no calculation was performed; the EEG data acquired just after the time window for a given time point is being used for the following time point, a new time window opens after one time window closes).

After receiving the EEG data, alpha and delta waves frequency information is extracted therefrom. The goal of this step is to obtain an information relevant as to the relative distribution of these waves in the EEG signal, to be able to compare them, increase of delta waves as compared to alpha waves being indicative of brain hypoperfusion.

The raw EEG signal (or multiple EEG signals when multiple electrodes have been positioned on the subject) can be filtered to isolate the alpha and delta frequency bands, in particular using a bandpass filter, which allows signals within the requested frequency range (7.5-13 Hz for alpha, 0.5-4 Hz for delta) to pass through while attenuating signals outside of that range. One can use any available type of filters, such as Butterworth or Chebyshev filters. One of skill in the art can select the appropriate filter design, such as the desired cutoff frequency and steepness of the filter. The cutoff frequencies and filter order are then designed, in order to encompass the alpha frequency band (7.5-13 Hz) and/or the delta frequency band (0.5-4 Hz). The bandpass filter is then applied to the raw EEG signal. It is also possible to evaluate the quality of the filtered signal to ensure that it allows further analysis, and adjustments to the filter parameters or use of different filter designs can be envisaged if needed.

Once information pertaining to the alpha and delta waves is extracted, one can use any parameter that is associated with the amount of alpha and delta waves as the alpha and delta waves information to perform the methods herein disclosed. One can cite the mean amplitude of the alpha and delta waves.

However, it is preferred to use respectively the alpha power ($\alpha$BP) and the delta power ($\delta$BP) computed from the EEG power spectral density (PSD) extracted from the EEG data, as the alpha and delta waves information. In this embodiment, the raw EEG signal obtained during the time window is processed to obtain the PSD, and the alpha power and delta power are obtained by integrating the values between respectively 7.5-13 Hz (or 8.0-13 Hz) and 0.5-4 Hz (calculating the area).

Thus, in this embodiment, the alpha and delta waves frequency information is extracted by

- Filtering EEG data
- Computing EEG power for each electrode using the fast Fourier Transform (FFT) to obtain EEG power spectral density (PSD)
- Optionally averaging the PSD for the multiple electrodes is multiple electrodes are used, to obtain a final PSD
- Obtaining alpha power ($\alpha$BP) and the delta power ($\delta$BP) by integrating the EEG power spectral density respectively across the 7.5-13 Hz (or 8.0-13 Hz) and 0.5-4 Hz values.

It is reminded that the power spectral density is a measure of the power distribution across different frequencies in an EEG signal. Although one of skill in the art is aware of several methods to calculate PSD from EEG data, a widely used method is the Welch's method.

The EEG signal would be processed, in particular by filtering the data to remove frequencies outside the range of interest, removing or interpolating bad channels, and detrending or normalizing the data. As the studied signal is obtained from a limited time window (an epoch, as indicated above), a window function, such as the Hamming, Hanning, or Blackman window, is applied to reduce spectral leakage and improve the spectral resolution of the PSD estimate.

The Fast Fourier Transform (FFT) can then be applied to each windowed epoch to estimate the power spectrum. The power spectrum is the magnitude squared of the complex FFT coefficients, and represents the distribution of power across different frequencies.

Other methods could be envisaged for analyzing EEG data besides FFT, such as wavelet analysis, Hilbert-Huang transform, or event-related spectral perturbation (ERSP) analysis, which involves calculating a baseline spectral power for a given frequency band (for instance, the baseline could be the values just before induction of anesthesia), and comparing this baseline to the spectral power during anesthesia.

When multiple electrodes are used to acquire the EEG data, the PSD calculated for each electrode can then be averaged to obtain the final PSD which is used to compute the alpha power ($\alpha$BP) and delta power ($\delta$BP).

The DAR (delta / alpha ratio) for the given time point can then be calculated by dividing the value obtained for delta waves by the equivalent value obtained for alpha waves. In particular, the DAR for a given time point is the result obtained by dividing the delta power by the alpha power.

As indicated above, when the EEG data comprises multiple EEG signal acquired on multiple electrodes, the alpha and delta waves frequency information is extracted from each EEG signal, and such information is averaged so that the DAR is calculated with the average of the alpha and delta waves frequency information.

The methods herein disclosed are based on the fact that the DAR will increase in case of brain hypoperfusion. The calculated DAR shall then be compared with the DAR calculated at the preceding time point to determine such increase or not. However, there is may be some variability between two DARs calculated at consecutive time points, which would not be relevant from a clinical point of view. For this reason, it is thus preferred to smooth the DAR data and to calculate the DAR to be compared as the average of the DARs computed from the given time point and consecutive preceding time points (preferably using 3 to 10, preferably 5 to 7, more preferably 6 preceding time points). The comparison is thus performed for DARs that are moving average. For instance, at a time point t, one shall calculate the DAR to compare by averaging the DARs computed at times t, t-1, t-2, t-3, t-4, t-5, and compare it with the average of the DARs computed at times t-1, t-2, t-3, t-4, t-5, t-6. At time t+1, one takes the average of DARs computed at times t+1, t, t-1, t-2, t-3, t-4 and compare it with the DAR of time t which is the average described above.

Alternatively, one can smooth the DAR plots using any regression method, such as Locally estimated scatterplot smoothing (LOESS, a nonparametric method which uses local regression to fit a smooth curve through a scatterplot of data).

In summary, the DAR calculated at c) of the method for a given time point and compared at d) can be:

- The DAR obtained with the values of the alpha and delta power computed for the time point
- An average of DARs obtained at the given time point and at 3-10 preceding time points. In this embodiment, one compares a moving average.

- The extrapolated value of a DAR as calculated from a local regression or smoothing (such as the LOESS), using the DAR obtained at the given time point and the previously obtained DARs.

The inventors were able to show that there is a delay between the decrease in blood cerebral flow (indicating hypoperfusion) and the increase of the DAR. Such delay is about 5-7 minutes, so that increase of the DAR indicates that brain hypoperfusion occurred between 5 to 7 minutes earlier.

The methods herein disclosed are of particular interest for anesthesia that may last more than one hour and/or for patients that could be classified at risk. In particular, the methods can be performed for anesthesia of a patient classified as having an ASA Physical Status equal or higher than 2, according to the ASA Physical Status Classification System. It is reminded that ASA is the American Society of Anesthesiologists (asahq.org). The method may also be performed for patients aged 50 or higher.

It is reminded that the ASA Physical Status Classification System comprises the following classification.

ASA I: A normal healthy patient (Healthy, non-smoking, no or minimal alcohol use, healthy (no acute or chronic disease), normal BMI percentile for age).

ASA II: A patient with mild systemic disease (Mild diseases only without substantive functional limitations. Current smoker, social alcohol drinker, pregnancy, obesity (30<BMI<40), well-controlled DM/HTN, mild lung disease, Asymptomatic congenital cardiac disease, well controlled dysrhythmias, asthma without exacerbation, well controlled epilepsy, non-insulin dependent diabetes mellitus, abnormal BMI percentile for age, mild/moderate OSA, oncologic state in remission, autism with mild limitations, Normal pregnancy, well controlled gestational HTN, controlled preeclampsia without severe features, diet-controlled gestational DM).

ASA III: A patient with severe systemic disease (Substantive functional limitations; One or more moderate to severe diseases. Poorly controlled DM or HTN, COPD, morbid obesity (BMI ≥40), active hepatitis, alcohol dependence or abuse, implanted pacemaker, moderate reduction of ejection fraction, ESRD undergoing regularly scheduled dialysis, history (>3 months) of MI, CVA, TIA, or CAD/stents. Uncorrected stable congenital cardiac abnormality, asthma with exacerbation, poorly controlled epilepsy, insulin dependent diabetes mellitus, morbid obesity, malnutrition, severe OSA, oncologic state, renal failure, muscular dystrophy, cystic fibrosis, history of organ transplantation, brain/spinal cord malformation, symptomatic hydrocephalus, premature infant PCA <60 weeks, autism with severe limitations, metabolic disease, difficult airway, long term parenteral nutrition. Full term infants <6 weeks of age. Preeclampsia with severe features, gestational DM with complications or high insulin requirements, a thrombophilic disease requiring anticoagulation).

ASA IV: A patient with severe systemic disease that is a constant threat to life (Recent (<3 months) MI, CVA, TIA or CAD/stents, ongoing cardiac ischemia or severe valve dysfunction, severe reduction of ejection fraction, shock, sepsis, DIC, ARD or ESRD not undergoing regularly scheduled dialysis Symptomatic congenital cardiac abnormality, congestive heart failure, active sequelae of prematurity, acute hypoxic-ischemic encephalopathy, shock, sepsis, disseminated intravascular coagulation, automatic implantable cardioverter-defibrillator, ventilator dependence, endocrinopathy, severe trauma, severe respiratory distress, advanced oncologic state. Preeclampsia with severe features complicated by HELLP or other adverse event, peripartum cardiomyopathy with EF <40, uncorrected/decompensated heart disease, acquired or congenital).

ASA V: A moribund patient who is not expected to survive without the operation (Ruptured abdominal/thoracic aneurysm, massive trauma, intracranial bleed with mass effect, ischemic bowel in the face of significant cardiac pathology or multiple organ/system dysfunction. Massive trauma, intracranial hemorrhage with mass effect, patient requiring ECMO, respiratory failure or arrest, malignant hypertension, decompensated congestive heart failure, hepatic encephalopathy, ischemic bowel or multiple organ/system dysfunction. Uterine rupture).

When the DAR at a given time point (whether directly calculated, moving average or interpolated by regression such as LOESS) is higher than the DAR at the preceding time point, it is preferred when a signal (a visual and/or sound signal) is emitted to alert the anesthetist of the occurrence of a potential hypoperfusion. Upon verification of other variables (such as mAP), the physician can decide to modify the anesthesia parameters, in particular to administer a vasopressor to increase mAP to increase brain perfusion. In order to avoid false positive signals, it is possible to emit the signal when the DARs at two consecutive time points are higher than the DARs of the preceding time points, or when the variation of the DAR (as compared to a reference DAR) is more than 10% (i.e. the obtained DAR at the given time point is at least 10% higher than the reference DAR).

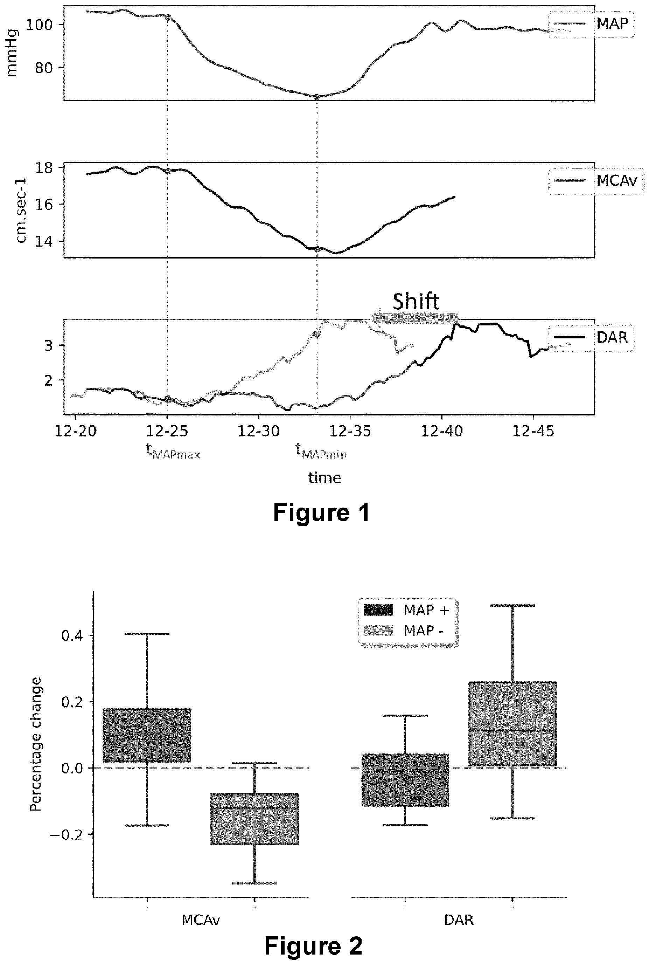

The reference DAR is the DAR obtained just after anesthesia has been established as a stable regimen. Generally, such stable anesthesia is obtained between 8-10 minutes after induction of anesthesia. It is indeed considered that the brain is well perfused at the beginning of the anesthesia, thus providing the reference DAR. As shown in Figure 2, one can indeed see that the DAR doesn't actually change when the mAP increases (which leads to an increase of blood velocity in the middle cerebral artery, MCAv), while the DAR increases when mAP and MCAv decrease.

In addition, and to ensure patient safety, a signal may be emitted when the mAP value is below a pre-determined threshold (this may be considered if the mean arterial pressure is below 65 mmHg). Such a signaling step can be incorporated into a method as described above. The signal can be a graphical signal (such as a representation of the mean arterial pressure by a color code different from the conventional color code (red in case of an alert instead of green or yellow). Alternatively, the value may be displayed with different color codes on the monitoring monitor to alert the physician to a risk of hypotension.

The alert signal can also or alternatively be an audible signal (long beep or other) when the mean arterial pressure falls below a predetermined value. This also alerts the surgeon of a problem, and of the need for the anesthetist to perform the medical procedure to correct this hypotension.

The method herein disclosed may further comprise monitoring the Mean Arterial Pressure (mAP) of the patient. Such monitoring may be performed using another device than the device used to monitor and/or analyze the EEG. The mAP is thus monitored and displayed on another monitor.

In some cases, mean arterial pressure is measured with a sphygmomanometer (or electronic electrocardioscope tensiometer) which uses the oscillometric measurement when the sleeve placed on the patient's forearm deflates automatically. The maximum value of the oscillation represents the mean arterial pressure. In other cases, one can use an intraarterial catheter that directly measures the mAP. However, this technic is very invasive and is generally not used in routine.

In some cases, mAP is measured using a plethysmograph (pulse oximeter located at the tip of a finger). WO 2017/037369 and WO 2007/128518 disclose such ways of measuring mAP.

In a specific embodiment, the mAP is continuously evaluated, based on the value of the height of the dicrotic wave, continuously calculated by plethysmography, using a method comprising:

I. calculating a calibration value Calib from

(a) the value of the mean arterial pressure measured at a time t0, and
(b) the height of the dicrotic wave Vp0 measured at the time t0, wherein

$$Calib = (\text{value of the mean arterial pressure measured at a time t0}) \, / \, Vp0$$

and

II. calculating the estimated value MAPest of the subject's mean arterial pressure at a time t after t0 by the formula MAPest = Calib x Vpt, wherein Vpt is the value of the dicrotic wave height obtained at the time t.

Such method for measuring mAP is disclosed in WO2019122406 and US20200390347.

As mentioned above, the methods described are of particular interest for monitoring a patient's brain perfusion continuously when the patient is under general anesthesia. This enables the physician to act quickly if the brain perfusion is too low (although taking into account the delay in the EEG data and the lowering of cerebral blood flow). This would reduce the risk of observing post-operatory side effects, avoid intraoperative brain awakening, and maintain sufficient perfusion to ensure brain cellular function and neuron protection.

Thus, it is preferred when these methods are used continuously throughout the duration of a patient's general anesthesia.

The methods herein disclosed also make it possible to perform a method for treating a subject (patient) in need thereof, comprising performing the method of analyzing the EEG of the patient, as disclosed above to detect hypoperfusion of the brain, and administering a vasopressor to the subject when hypoperfusion of the brain is detected. The vasopressor increases arterial pressure. Vasopressors are known in the art and include sympathomimetics (including adrenaline, dopamine, ephedrine...), glucocorticoids and mineralocorticoids, angiotensinamide... In hospital settings, sympathomimetics are used instead.

The invention also relates to a vasopressor for use for increasing mAP and/or brain cerebral flow (brain perfusion) in a patient when the method as applied to the patient indicated hypoperfusion of the brain.

The invention also relates to a system for monitoring brain perfusion anesthesia of a subject, wherein the subject is in a state of stable/stationary anesthesia, comprising

a. first means for storing the electroencephalogram data of the subject, wherein the first mean provides the alpha and delta waves of the subject [database]
b. second means for calculating the delta / alpha ratio at a time t (including a [processor for cutting the data into consecutive time windows, performing FFT, integrating between the alpha and delta frequencies, calculating the

6

DAR, storing the DAR with the time of calculus in a database)

c. third means for comparing the calculated delta / alpha ratio at the time t to the delta / alpha ration calculated at the preceding time t-1 (such as extracting the DAR previously calculated and comparing the values of DAR(t) and the DAR(t-1) [difference or ratio], or extracting n values of previously stored DAR for previous consecutive time point, calculating the mean of these extracted values and of the value of DAR(t) and comparing the mean to the mean for the n+1 previously calculated DAR before t)

d. means for displaying a signal when the calculated delta / alpha ratio at the time t is lower to the delta / alpha ration calculated at the time t-1 and/or means for displaying the DAR and its variation (including percentage of variation)

The system thus contains:

- a first database where the EEG received from one or multiple electrodes is stored as such. This stored data can be linked to information pertaining to the patient (that could be stored in another database) such as name or social security number or other identification information, and characteristics (such as age, height, weight, BMI, comorbidities...), as well as other relevant information (such as date and/or time of operation)
- a processor for processing the stored EEG and calculating DAR for each time point. The processor thus peforms

  ∘ cutting the stored EEG data in epochs of a set-up time window
  ∘ extracting the frequency information from the EEG data
  ∘ extracting the information for alpha and delta waves
  ∘ determining the value for alpha and delta waves that is to be used for the DAR calculation
  ∘ optionally averaging such values when EEG data from multiple electrodes is stored in the database
  ∘ optionally storing the alpha and delta waves information
  ∘ calculating the DAR from the alpha and delta waves values
  ∘ storing the DAR in a database together with time information to have a (DAR, epoch/time point) relation in the database

- a processor (which may be the same) for comparing the DAR from one time point to the DAR from preceding time point(s). This is done by

  ∘ extracting, from the database defined above, the DAR calculated for at least the preceding time point, and optionally for more than one preceding time point
  ∘ if more than one DAR was extracted, obtaining the mean of the multiple extracted DAEs
  ∘ comparing the DAR calculated at one time point to the DAR of the preceding time point or to the mean of multiple extracted DARs; this is performed by This can be done by *via* division or subtraction.
  ∘ optionally storing the comparison in a database, together with the time point on which it was calculated

- optionally a screen to display the DAR calculated for each time point (DAR on the ordinate, and time on the abscissa) overtime, and optionally a processor to display a regression curve calculated (and recalculated) every time a new DAR is calculated
- preferably means to display a signal (visual and/or sound signal) when the DAR increases over time (according to parameters entered by the physician (percentage of increase, duration of increase...)
- optionally means for the automatic administration of a dose of vasopressor, in the event that the method provides for an increase in the DAR, indicative of a low brain perfusion.

The system or device may also contain means for receiving the EEG of a subject, from one or multiple electrodes placed in the patient's head.

The invention also relates to a computer product/program comprising program code instructions recorded on a computer-readable medium, for carrying out the steps of the methods described above, when said program is executed on a computer.

This program may also contain code instructions to display the DAR values on a monitor. It may also include code instructions to emit a visual and/or audible signal if the calculated DAR variation is above a predetermined threshold (preprogrammed or entered by the clinician). Indeed, one can consider that the DAR variation is to be considered only if it is (in percentage) higher than a predetermined percentage (as compared to a baseline DAR, such as the one calculated at the induction of anesthesia), or if there is a trend of increase of DSAR (increase for more than two, three or more consecutive time points).

The invention also relates to a computer-readable recording medium on which is recorded a computer program comprising program code instructions for carrying out the steps of processes as described above or programs as described above.

The interest of the present methods and devices is that, by looking at the DAR variation, it is possible to detect brain hypoperfusion for a given patient, even if the mAP remains within the standard recommendations (60-65 mmHg pour patients without comorbidities, 70-75 mmHh for patients with comorbidities in particular cardiovascular problems). For these patients, the Lassen curve would lead the physician to believe that there is a proper blood perfusion.

This allows the anesthetist to provide vasopressor to increase the mAP (and hence brain perfusion), while such action would not have been made but only looking at the mAP value. The methods and devices also allow the anesthetist not to provide vasopressor in case of decrease of the mAP if the DAR doesn't increase: this would indeed indicate that the brain remains sufficiently perfused, despite the mAP decrease.

Using the methods and devices herein disclosed, the physician can thus more finely adjust the patient's mAP and administration of vasopressor in stable anesthesia to increase patient's wellness.

## FIGURES

Figure 1: Percentage change of mAP calculated from the maximum and the minimal value detected during stable anesthesia (upper graph). Min and max mAP values happen at times tMAPmin and tMAPmax, respectively (dashed, vertical lines). The percentage change in MCAv (time averaged maximum blood velocity in the middle cerebral artery) was calculated using the MCAv value corresponding to the tMAPmax and tMAPmin time values; Optimal EEG lag was identified by shifting the Delta-to-Alpha ratio (DAR) until correlation was maximal. DAR time series was computed using 10-second long EEG epochs.

Figure 2: Comparing distributions of change in MCAv and DAR between positive and negative MAP variations. Percentage change of MCAv and DAR in the group with positive induced MAP variations (MAP +, blue) and the group with negative variations (MAP -, orange). The star represents significant difference by One-sample Wilcoxon test.

## EXAMPLES

## Example 1. Methods

**Patients:** Patients eligible for interventional neuroradiology surgery under general anesthesia were selected for the "cervo2" cohort, a prospective, observational, mono-centric study conducted at Lariboisière Hospital in Paris, France, according to appropriate ethical guidelines and with informed consent. Subsequently, between February 2021 and November 2022, patients eligible for surgery under general anesthesia were selected for the "delta PAM" cohort, a prospective, interventional, mono-centric study also carried out at Lariboisière Hospital, according to appropriate ethical guidelines and with informed consent..

Inclusion criteria were elective surgery, propofol intravenous anesthesia, and being a French-speaking adult (>18yo.). Exclusion criteria encompassed pregnant women, patients with a history of a bleeding aneurysm (incidental finding of aneurysm following either cephalea or tinnitus complaints), emergency procedures for subarachnoid hemorrhage, and individuals with a Body Mass Index (BMI) > 35 kg/m$^2$, for whom the Schnider's model is not validated. Patient demographics were collected during the anesthesia consultation. Doppler signal was measured on the side opposed to the location of the aneurysm. Patients were considered to have cardiovascular risk factors if they presented with any of the following criteria: history of a cardiovascular event, current smoking, diabetes mellitus, dyslipidaemia, or arterial hypertension.

**Anesthesia protocol:** Standard monitoring, including monitoring of pulse oxygen saturation (SpO$_2$), heart rate (HR), systolic (sBP), diastolic (dBP) and mean (mAP) arterial blood pressure, temperature, end-tidal CO$_2$ (EtCO$_2$), was conducted alongside electroencephalogram (EEG) monitoring using the Masimo Sedline monitor and metrics including SEF95, Patient State Index (PSI), and burst suppression ratio (BS). Total intravenous anesthesia (TIVA) was administered in a standardized manner, starting with remifentanil (Minto's model) followed by Propofol (Schnider's model). Atracurium besilate (0.5 mg.kg$^{-1}$) was used to induce paralysis, after which all patients were intubated and mechanically ventilated with a tidal volume of 6-8 ml.kg$^{-1}$, respiratory rate adjusted to achieve an EtCO$_2$ of 35-38 mmHg, and an FiO2 set at 40%. The temperature was maintained between 36°C and 37°C.

Remifentanil target controlled infusion (TCI) was initially set to 5-6 ng.ml$^{-1}$ until oral tracheal intubation, then reduced for maintenance to 2.5-3.5 ng.ml$^{-1}$. However, the anesthesiologist could adjust infusion rates at any time to maintain stable hypnosis, as indicated by the absence of burst suppression and a PSI of 25-50.

**mAP challenge:** The implementation of a mAP challenge was carried out within the commonly used value ranges, considering the patient's cardiovascular comorbidities. Currently, a mAP greater than 60 mmHg and a reduction of less than 30 to 50% from the value measured before general anesthesia are widely used treatment thresholds to ensure adequate perfusion of all organs.

Diluted norepinephrine at 5μg/ml was the only vasoconstrictor used, administered either by a 10μg bolus or continuously through intravenous injection using syringe pumps, with a rate set to maintain mAP above the hypotension threshold. Patients who experienced a hypotensive episode following induction were resuscitated with a 10μg bolus of diluted norepinephrine at 5μg/ml, immediately followed by a continuous infusion of diluted norepinephrine at 5μg/ml at a rate of 40 mL/hour (i.e. 200 μg/hour or 10 μg/10min). If a continuous infusion was already ongoing, an additional increase of 40 ml/hour was added.

In case mAP was ≥ 90% of the value measured during the anesthesia consultation, the dose of norepinephrine was gradually decreased to reach a mAP ≥ 50-70% of the baseline value, without ever going below 60 mmHg for patients without cardiovascular risk factors and 80 mmHg for patients with cardiovascular risk factors. Only one mAP challenge per patient was analyzed resulting in two groups of patients: a group in which the impact of increasing mAP was analyzed (mAP +) and a group in which the impact of a decreasing mAP was analyzed (mAP-).

**Data acquisition**

*Blood pressure recording based on photoplethysmography*: Intraoperative arterial blood pressure was continuously monitored, starting before anesthesia, from the middle finger using Clearsight® device (Edwards Lifesciences, Irvine, CA). The analog signal was transmitted to IntelliVue MP60 monitor (Philips, Eindhoven, The Netherlands) at a sampling rate frequency of 125Hz.

*Blood flow velocity measured using transcranial Doppler*: Blood flow velocity in middle cerebral artery was measured using a 1.5MHz ultrasound Doppler probe placed on one side of the head, always contralateral to the intervention site. The probe could not remain in this position during the neuroradiology procedure and was only set there during the patient's anesthesia induction and for the subsequent 30 minutes. Probe power was set at 100mW, the gate at 9mm for a 45-55mm depth, and a gain ranging from 2 to 4. The analog signal was transmitted to IntelliVue MP60 monitor at a 125Hz sampling rate frequency.

*EEG measurements:* Bilateral frontal EEG traces (Fp1, Fp2, F7, F8, with a ground electrode Fpz and a reference) were collected from Masimo Root® Sedline® monitor (Masimo Corporation©, Irvine, California, USA) at a recording frequency of 89Hz. PSI was transmitted to IntelliVue MP60 monitor at a 1Hz sampling rate frequency. The traces were first exported in the *.edf* format, and then the EEG traces were concatenated and analyzed using scipy, statsmodel, and mne Python libraries.

**Feature extraction from EEG traces:** The EEG was recorded throughout the entire intervention; however, EEG features were computed only when the plasma concentration of propofol remained constant for more than 800 seconds, thereby indicating stable anesthesia. The EEG signal was divided into non-overlapping epochs, each 10s long. A time series of EEG features was generated by calculating a value for each epoch.

To compute the power of the different frequency bands, for each epoch and electrode, the power spectral density (PSD) was calculated using Welch's method with a window of 512 points (approximately 5.75s) and a 50% overlap, applying a Hamming window. The PSD was then averaged over the four EEG electrodes.

The power of each band was computed by integrating the PSD over the corresponding frequencies. The total power was obtained by integrating the PSD over the full range of frequencies (0.5-30) Hz. From these powers, we computed the Delta-alpha ratio (DAR), defined as the ratio of alpha power ($\alpha$BP) to delta power ($\delta$BP). The alpha peak frequency ($\alpha$PF) corresponded to the frequency at which the PSD reached its maximum in the alpha band (8-13)Hz, for which the value was estimated for each epoch.

**Intraoperative time series pre-processing** :

During the EEG trace recording, simultaneous measurements of mAP and the mean blood velocity in the mean cerebral artery (MCAv$_{mean}$) were taken. To align with the 10-second sampling frequency of the EEG features, these physiological data were preprocessed prior to analysis. This preprocessing involved dividing the original time series into 10-seconds long epochs, and then calculating the time-averaged values within each epoch. Thus, the mean cerebral artery (MCAv$_{mean}$) was defined as the within epochs time averaged maximum velocity measured using transcranial Doppler. Mean arterial Pressure (mAP) was defined as the within epochs time averaged arterial pressure recorded with photoplethysmography.

To estimate slow physiological trends on a minute scale, mAP, MCAv$_{mean}$, and $\alpha$PF time series were smoothed by applying two successive 50-second long moving averages. The features including $\alpha$BP, $\delta$BP, SEF95, DAR, and $\alpha$PF were smoothed using Locally Weighted Scatterplot Smoothing (LOWESS, with a fraction of 5% of the data used when estimating each y-value).

For each patient (Fig. 1), the percentage change in mAP was calculated based on the maximum and minimum values detected during stable anesthesia; the percentage change in MCAv was calculated based on the MCAv value corresponding to the time when maximum and minimum values of mAP (tMAPmax, tMAPmin) were observed; multiple percentage changes in EEG markers were calculated based on the EEG markers values corresponding to the times tMAPmax and tMAPmin, with lags ranging between 0 and 1200 seconds (20 minutes). This approach is illustrated in **fig. 1.**

**Data analysis:** Linear regression parameters ("slope", "intercept", "rvalue", "pvalue", "stderr") between percentage change of $MCAv_{mean}$ and percentage change of each EEG feature lagged between 0 seconds and 1200 seconds (= 20 minutes). Optimal population LAG was defined as the values of lag with the higher rvalue and significant p-value. Statistical analysis between the two groups were performed with EEG features lagged according to the optimal lag.

**Statistical analysis:** The significance level was 0.05. Data with normal distribution, assessed using a Shapiro-Wilk test, were shown as mean standard deviation, non-normal distribution (resp. categorical variables) were expressed by the median and interquartile range IQR (resp. as the count and percentage). Covariates were compared with Fisher, t-test or Mann-Whitney tests as appropriate.

## Example 2. Results

### Patients

93 patients were prospectively included and 55 matched criteria for further analysis (age mean=45 $\pm$ 15y.o., 71% women). The mean absolute percentage changes in mAP and CBF were 41 $\pm$ 20% and 14 $\pm$ 11%, respectively.

### Relationship between CBF changes and quantitative intraoperative EEG with incremental lag

A significant correlation between CBF and EEG percentage changes was found, especially considering the DAR (r = 0.51, p<0.001). The average latency between CBF and EEG response was of 380 seconds, which indicates that the DAR starts, in average, to increase 380 seconds after decrease of the CBF. DAR appeared as a robust marker because it was the only marker with a significant correlation.

### DAR increases in case of brain hypoperfusion (decrease of time averaged maximum blood velocity in the middle cerebral artery (MCAv) and remains stable in case of increase of time averaged maximum blood velocity in the middle cerebral artery

Either positive or negative mAP variations were pharmacologically induced between two recommended thresholds. CBF was approximated by the time averaged maximum blood velocity in the middle cerebral artery (MCAv) measured using transcranial Doppler.

Table 1. mean absolute percentage changes in mAP and CBF

| absolute values | All (n=55) | MAP + (n=37) | MAP - (n=18) | p-value |
|---|---|---|---|---|
| d mAP (%) | 43 (18) | 40 (20) | 43 (16) | 0.67 |
| d MCAv (%) | 13 (14) | 11 (13) | 15(11) | 0.26 |
| time (min) | 6.6 [3.5-8] | 6.1 [2.6-8.5] | 7.6 [4.2-10.3] | 0.34 |

Figure 2 shows that an increase of mAP induced an increase in blood velocity in the middle cerebral artery, but no significant difference in the DAR.

However decrease of mAP induced a decrease in blood velocity in the middle cerebral artery, and an increase of the DAR.

It is thus supposed that, when the blood is well perfused, increase of mAP (hyperperfusion) doesn't change the brain activity, when hypoperfusion of the brain induces physiological changes that are detected by the DAR as herein disclosed.

Conclusion: Changes in CBF following modulation of the MAP affect the EEG during general anesthesia. This response is not synchronous but delayed and well captured using the delta-alpha ratio variable.

## Claims

**1.** A ex *vivo* method for continuously monitoring brain perfusion during anesthesia of a subject, wherein the patient is in a state of stable/stationary anesthesia, comprising

a. Receiving, at consecutive time points, data from an electroencephalogram (EEG) of the subject, wherein this data comprises the cerebral activity recorded during a time window preceding a given time point
b. Extracting alpha and delta waves frequency information
c. Calculating delta / alpha ratio (DAR) using the information of b), wherein the DAR is the ration of the power of the alpha and delta waves
d. Comparing the calculated DAR to the DAR calculated at the preceding time point,

wherein increase of the calculated DAR indicates hypoperfusion of the brain of the subject.

2. The method of claim 1, wherein the anesthesia is considered as stable/stationary when the rate of administration of anesthetic drugs is stable.

3. The method of claim 1 or 2, wherein the time points are spaced apart by a duration corresponding to the time window.

4. The method of any one of claims 1 to 3, wherein the alpha and delta frequency information are respectively the alpha power ($\alpha$BP) and the delta power ($\delta$BP) computed from the EEG power spectral density extracted from the EEG data.

5. The method of any one of claims 1 to 4, wherein the time window for recording the EEG signal is comprised between 5 and 30 seconds, preferably 5 and 20 seconds, preferably 10 seconds.

6. The method of any one of claims 1 to 5, wherein the EEG data comprises multiple EEG signal acquired on multiple electrodes, wherein the alpha and delta waves frequency information is extracted from each EEG signal, and wherein the DAR is calculated with the average of the alpha and delta waves frequency information.

7. The method of any one to claims 1 to 6, wherein the calculated DAR is an average of the delta / alpha ratio computed from multiple consecutive time points, in particular from 3 to 10 consecutive time points, , preferably from 5 to 7 consecutive time points, preferably from 6 consecutive time points.

8. The method of claim 4, wherein the alpha and delta waves frequency information is extracted by

- Filtering EEG data
- Computing EEG power for each electrode using the fast Fourier Transform (FFT) to obtain EEG power spectral density
- Obtaining alpha power ($\alpha$BP) and the delta power ($\delta$BP) by integrating the EEG power spectral density respectively across the 8.0-13 Hz and 0.5-4 Hz values.

9. The method of any one of claims 1 to 8, wherein increase of the calculated DAR indicates that brain hypoperfusion occurred between 5 to 7 minutes earlier.

10. The method of any one of claims 1 to 9, wherein the patient is classified as having an ASA (American Society of Anesthesiologists) Physical Status equal or higher than 2, according to the ASA Physical Status Classification System.

11. The method of any one of claim 1 to 10, wherein emitting a signal is emitted when the DAR calculated at a given time point is higher than the DAR calculated at the preceding time point.

12. The method of any one of claims 1 to 11, further comprising monitoring the Mean Arterial Pressure (MAP) of the subject.

13. The method of claim 12, wherein the MAP is continuously evaluated, based on values of the height of the dicrotic wave continuously calculated by plethysmography, the method comprising:

I. calculating a calibration value Calib from

(a) the value of the mean arterial pressure measured at a time t0, and

(b) the height of the dicrotic wave Vp0 measured at the time t0, wherein

$$\text{Calib} = (\text{value of the mean arterial pressure measured at a time t0}) / \text{Vp0}$$

and

II. calculating the estimated value MAPest of the subject's mean arterial pressure at a time t after t0 by the formula MAPest = Calib x Vpt, wherein Vpt is the value of the dicrotic wave height obtained at the time t.

14. The method of any one of claims 1 to 13, which is implemented by a computer.

15. A system for monitoring brain perfusion anesthesia of a subject, wherein the patient is in a state of stable/stationary anesthesia, comprising

    a. first means for storing the electroencephalogram data of the subject within a database
    b. second means for calculating the delta / alpha ratio at a time t
    c. third means for comparing the calculated delta / alpha ratio at the time t to the delta / alpha ratio calculated at the preceding time t-1
    d. preferably means for displaying a signal when the calculated delta / alpha ratio at the time t is lower to the delta / alpha ratio calculated at the time t-1 and/or means for displaying the DAR and its variation (including percentage of variation)

**Figure 1**

**Figure 2**

Europäisches
Patentamt

European
Patent Office

Office européen
des brevets

# EUROPEAN SEARCH REPORT

**Application Number**

**EP 23 17 2461**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | AJCEVIC MILOS ET AL: "Early EEG Alterations Correlate with CTP Hypoperfused Volumes and Neurological Deficit: A Wireless EEG Study in Hyper-Acute Ischemic Stroke", ANNALS OF BIOMEDICAL ENGINEERING, SPRINGER US, NEW YORK, vol. 49, no. 9, 18 February 2021 (2021-02-18), pages 2150-2158, XP037568353, ISSN: 0090-6964, DOI: 10.1007/S10439-021-02735-W [retrieved on 2021-02-18] * the whole document * ----- | 1-15 | INV. A61B5/026 A61B5/374 |
| Y | US 2020/222008 A1 (JENSEN ERIK WEBER [ES] ET AL) 16 July 2020 (2020-07-16) * page 46; claim 1 * ----- | 1-15 | |
| A | CLAASSEN J ET AL: "Quantitative continuous EEG for detecting delayed cerebral ischemia in patients with poor-grade subarachnoid hemorrhage", CLINICAL NEUROPHYSIOLOGY, ELSEVIER, AMSTERDAM, NL, vol. 115, no. 12, 1 December 2004 (2004-12-01), pages 2699-2710, XP004645453, ISSN: 1388-2457, DOI: 10.1016/J.CLINPH.2004.06.017 * the whole document * ----- -/-- | 1,15 | **TECHNICAL FIELDS SEARCHED (IPC)** A61B |

The present search report has been drawn up for all claims

| Place of search | Date of completion of the search | Examiner |
|---|---|---|
| Berlin | 22 September 2023 | Kronberger, Raphael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 2**

**EUROPEAN SEARCH REPORT**

Application Number

EP 23 17 2461

## DOCUMENTS CONSIDERED TO BE RELEVANT

| Category | Citation of document with indication, where appropriate, of relevant passages | Relevant to claim |
|---|---|---|
| A | LOSKOTA ET AL: "Intraoperative EEG monitoring", SEMINARS IN ANESTHESIA, SAUNDERS, CO, NEW YORK, NY, US, vol. 24, no. 4, 1 December 2005 (2005-12-01), pages 176-185, XP005206611, ISSN: 0277-0326, DOI: 10.1053/J.SANE.2005.10.007 * the whole document * | 1,15 |
| A | R. L. O&RSQUO;GORMAN ET AL: "Coupling Between Resting Cerebral Perfusion and EEG", BRAIN TOPOGRAPHY, vol. 26, no. 3, 1 July 2013 (2013-07-01), pages 442-457, XP055159070, ISSN: 0896-0267, DOI: 10.1007/s10548-012-0265-7 * the whole document * | 1,15 |

**CLASSIFICATION OF THE APPLICATION (IPC)**

**TECHNICAL FIELDS SEARCHED (IPC)**

The present search report has been drawn up for all claims

| Place of search | Date of completion of the search | Examiner |
|---|---|---|
| Berlin | 22 September 2023 | Kronberger, Raphael |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 23 17 2461

This annex lists the patent family members relating to the patent documents cited in the above-mentioned European search report.
The members are as contained in the European Patent Office EDP file on
The European Patent Office is in no way liable for these particulars which are merely given for the purpose of information.

22-09-2023

| Patent document cited in search report | | Publication date | Patent family member(s) | | Publication date |
|---|---|---|---|---|---|
| US 2020222008 | A1 | 16-07-2020 | BR 112019024732 | A2 | 16-06-2020 |
| | | | CN 110740681 | A | 31-01-2020 |
| | | | EP 3638099 | A1 | 22-04-2020 |
| | | | JP 7189897 | B2 | 14-12-2022 |
| | | | JP 2020523113 | A | 06-08-2020 |
| | | | RU 2020100456 | A | 14-07-2021 |
| | | | US 2020222008 | A1 | 16-07-2020 |
| | | | WO 2018228813 | A1 | 20-12-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017037369 A
- WO 2007128518 A
- WO 2019122406 A
- US 20200390347 A

**Non-patent literature cited in the description**

- **BRASSARD et al.** *Physiol Rep.,* August 2021, vol. 9 (15), e14982